Europäisches Patentamt

European Patent Office

Office européen des brevets

(11) Publication number: **0 278 407 B1**

(12) **EUROPEAN PATENT SPECIFICATION**

(45) Date of publication of patent specification: **08.06.94**    (51) Int. Cl.⁵: **C07C 31/125**, C07C 29/16

(21) Application number: **88101641.4**

(22) Date of filing: **04.02.88**

The file contains technical information submitted
after the application was filed and not included in
this specification

(54) **Alcohol mixture for plasticizer.**

(30) Priority: **09.02.87 JP 27561/87**

(43) Date of publication of application:
**17.08.88 Bulletin 88/33**

(45) Publication of the grant of the patent:
**08.06.94 Bulletin 94/23**

(84) Designated Contracting States:
**DE FR GB NL**

(56) References cited:
**EP-A- 0 052 999**
**EP-A- 0 183 547**
**DE-B- 1 027 194**
**GB-A- 789 777**
**US-A- 4 443 638**

**PATENT ABSTRACTS OF JAPAN, unexamin-
ed applications, C section, vol. 3, no. 109,
September 12, 1979, THE PATENT OFFICE
JAPANESE GOVERNMENT, page 81 C 58**

(73) Proprietor: **MITSUBISHI KASEI CORPORATION**
**5-2, Marunouchi 2-chome**
**Chiyoda-ku**
**Tokyo 100(JP)**

(72) Inventor: **Miyazawa, Chihiro**
**1-200-605, Hiroe 3-chome**
**Kurashiki-shi Okayama-ken(JP)**
Inventor: **Orita, Souichi**
**1-71-12, Hiroe 3-chome**
**Kurashiki-shi Okayama-ken(JP)**
Inventor: **Tsuboi, Akio**
**1-A-2, Hiroe 3-chome**
**Kurashiki-shi Okayama-ken(JP)**

(74) Representative: **Wächtershäuser, Günter, Dr.**
**Tal 29**
**D-80331 München (DE)**

## Description

The present invention relates to an alcohol mixture for plasticizer. More particularly, the present invention relates to a $C_9$ alcohol mixture having excellent heat resistance, cold resistance and electrical insulating properties as an alcohol material for plasticizer for a vinyl chloride resin.

It is known that a $C_9$ alcohol mixture obtained by the hydroformylation and hydrogenation of a $C_8$ olefin mixture (hereinafter referred to as "octenes") as the starting material obtainable by the dimerization of a $C_4$ fraction (hereinafter referred to as a "butene fraction") composed mainly of butene which is obtainable in a large quantity by the thermal decomposition of naphtha or by the catalytic decomposition of heavy or light oil, can be suitably employed as a starting material for plasticizer for a vinyl chloride resin. It is also known that the $C_9$ alcohol mixture has excellent heat resistance and is useful as an alcohol material for plasticizer, as disclosed in UK Patent No. 789,777.

However, such a $C_9$ alcohol mixture is a mixture of many kinds of isomers since octenes obtainable primarily by the dimerization of the butene fraction include many isomers, and the properties of the plasticizer vary substantially depending upon the composition of these isomers, whereby a substantial difference will be brought about in the cold resistance, the electrical insulating properties, etc. as the important properties of a vinyl chloride resin.

Accordingly, improvement of the properties of the $C_9$ alcohol mixture is one of the most important subjects to those skilled in the art. However, because of the complexity, not very much has been known for the improvement of the properties. Especially, little has been known for a method of obtaining a $C_9$ alcohol mixture for plasticizer which is excellent in both the cold resistance and the electrical insulating properties.

JP-A-54-88206 describes a method for preparing an alcohol for a plasticizer wherein $C_8$ olefins, which are formed by dimerizing butene fractions, having a boiling point within a range of 114 to 125°C are subjected to hydroformylation to synthesize a $C_9$ alcohol, followed by distilling and purifying to obtain an alcohol having a boiling point within a range of 194 to 208°C. Low boiling point $C_9$ alcohols and high boiling point $C_9$ alcohols are removed by the distillation process. The properties of a plasticizer obtained by using the $C_9$ alcohol mixture described in the reference are unsatisfactory, particularily with respect to its insulation properties.

DE-A-10 27 194 describes the synthesis of $C_9$ alcohol by dimerization of butenes and hydroformylation and hydrogenation of the dimer and the synthesis of $C_7$-$C_9$ alcohol by dimerization of propylene and butylene and hydroformylation and hydrogenation of the dimer. The reference does not describe the constitution and effects of controlling $C_9$ alcohol isomers to a specific condition and the properties of the plasticizer obtained by using such mixture of $C_9$ alcohols.

EP-A-0 052 999 describes a method wherein a $C_8$-$C_{10}$ saturated alcohol mixture is obtained by conducting oxo reaction of a mixture of propylene and butenes, followed by subjecting the resultant to aldol condensation and hydrogenation.

In view of the above-mentioned conventional techniques, the present inventors have conducted extensive reseach to obtain an alcohol mixture for plasticizer which is excellent in both the cold resistance and the electrical insulating properties and as a result, have found that a $C_9$ alcohol mixture obtained by hydroformylating octenes obtainable by the dimerization of a butene fraction, followed by hydrogenation, which has a specific distribution of the isomers such that when the isomers are divided into certain specific three group components, the proportions of the respective group components are within certain specific ranges, exhibits excellent performance in both the cold resistance and the electrical insulating properties when used for plasticizer. The present invention has been accomplished on the basis of this discovery.

Namely, the object of the present invention is to provide a $C_9$ alcohol mixture exhibiting excellent performance in the cold resistance, the heat resistance and the electrical insulating properties as the alcohol material for plasticizer for a vinyl chloride resin.

In the broadest sense, the present invention provides a $C_9$ alcohol mixture for plasticizer obtained by the hydroformylation and hydrogenation of a $C_8$ olefin mixture as the starting material obtained by the dimerization of a butene fraction, said alcohol mixture comprising from 10 to 50% by weight of a first group component having a retention time not longer than the retention time of isobutyl n-caprylate (i.e. octanoic acid isobutyl ester), from 10 to 40% by weight of a second group component having a retention time longer than that of isobutyl n-caprylate and not longer than that of methyl n-caprate (i.e. decanoic acid methyl ester) and from 30 to 70% by weight of a third group component having a retention time longer than that of methyl n-caprate, as divided into such three components by gas chromatography using a capillary column packed with a polyethylene glycol having a number average molecular weight of 15,000 to 20,000 as an isomer separating agent.

Now, the present invention will be described in detail with reference to the preferred embodiments.

The butene fraction as the starting material for the alcohol mixture for plasticizer of the present invention may be a $C_4$ fraction (hereinafter referred to as a "BB fraction") obtained by the thermal decomposition of naphtha or a BB fraction obtained by the catalytic decomposition of heavy or light oil.

The BB fraction obtained by the thermal decomposition of naphtha may directly be dimerized. However, a so-called spent BB fraction obtained by separating butadiene out of the BB fraction by extraction, or a so-called spent spent BB fraction obtained by further separating isobutene out of the spent BB fraction, may also be suitably employed.

The BB fraction obtained by the catalytic decomposition of heavy or light oil is a mixture composed mainly of butene and butane. This fraction may be directly dimerized for use. However, a BB fraction obtained by separating isobutene out of such a fraction by e.g. distillation may also be suitably used.

Such a butene fraction can be dimerized by a usual dimerization reaction. For example, the dimerization may be conducted by a so-called Ziegler catalyst method wherein a nickel salt and an aluminum-alkyl halide are used as the catalyst, or by an acid catalyst method wherein solid phosphoric acid is used as the catalyst.

The $C_8$ olefin mixture obtained by the dimerization of the BB fraction comprises various isomers, all of which are useful as the starting material for the hydroformylation reaction.

The hydroformylation reaction can be conducted by a conventional method. As the catalyst, any catalysts commonly employed for the hydroformylation reaction on an industrial scale, such as a rhodium catalyst or a cobalt catalyst, may be employed.

The hydroformylation reaction using rhodium as the catalyst is usually conducted under reaction condition such that the rhodium concentration is from 0.1 to 100 ppm as rhodium atom, the temperature is from 80 to 200°C, the pressure is from atmospheric pressure to 490 bar (500 kg/cm$^2$G) (1 kg/cm$^2$G = 0.980665 bar) and the $H_2$/CO volume ratio is from 0.5 to 4. Further, the rhodium catalyst may be employed as modified by a ligand such as a triarylphosphine (e.g. triphenylphosphine). No solvent is usually required. However, it is possible to use an organic solvent inert to the reaction.

The hydroformylation reaction using cobalt as the catalyst, is usually conducted under a reaction condition such that the cobalt concentration is from 0.05 to 10% by weight as cobalt atom, the temperature is from 80 to 180°C, the pressure is from 49 to 294 bar (50 to 300 kg/cm$^2$G) and the $H_2$/CO volume ratio is from 0.5 to 4. Further, the cobalt catalyst may be used as modified by a ligand such as a triarylphosphine (e.g. triphenylphosphine). No solvent is usually required. However, it is possible to employ an organic solvent inert to the reaction.

The $C_9$ aldehyde mixture or a mixture of $C_9$ aldehydes and $C_9$ alcohols obtained by the above hydroformylation reaction of the $C_8$ olefin mixture, is usually converted to $C_9$ alcohol mixture by a hydrogenation reaction. The hydrogenation reaction is usually conducted under a pressure of at least atmospheric pressure, preferably from 30 to 300 atm, at a temperature of at least room temperature, preferably from 100 to 200°C, by using a usual hydrogenation catalyst such as nickel, chromium or copper.

The crude alcohol mixture thus obtained is then purified by distillation in a rectification tower. The rectification of the crude alcohol mixture is usually conducted by using a distillation tower having a theoretical number of plates of from 3 to 50 plates under a condition such that the tower top pressure is from a few mmHg to 760 mmHg and the tower top temperature is from 50 to 220°C.

The alcohol mixture for plasticizer of the present invention may readily be obtained by a method wherein the amount of the distillate during the rectification is controlled to obtain the composition within the range of the present invention, or by a method wherein the distillate is finely classified and the classified fractions are suitably mixed to obtain the composition of the present invention.

The composition of the alcohol mixture for plasticizer thus obtained will be determined as follows. Firstly, by using a polyethylene glycol having a number average molecular weight of from 15,000 to 20,000 as an isomer separating agent (packing agent), the alcohol mixture obtained as above is analyzed by gas chromatography using a capillary column packed with the packing agent, and the detected isomer components are divided into three group components based on the retention times of two internal standard substances (isobutyl n-caprylate and methyl n-caprate). Namely, the first internal standard substance is isobutyl n-caprylate, and the second internal standard substance is methyl n-caprate.

The analytical condition by the gas chromatography using a capillary column packed with the above-mentioned packing agent, is for example as follows.

(1) Column: Packing "polyethylene glycol 20M" (manufactured by Gasukuro Kogyo Inc.), FFS (flexible fused silica) capillary column having a wall thickness of 0.15 $\mu$m, a length of 50 m and a diameter of 0.25 mm.

(2) Detector: FID (hydrogen flame ionization detector).

3

(3) Carrier gas: Helium gas, capillary: 0.66 ml/min,
purge: 43.56 ml/min.

(4) Combustion gas: Hydrogen: 0,59 bar (0.6 kg/cm$^2$G)
Air: 0.78 bar (0.8 kg/cm$^2$G)

(5) Temperature conditions:

Initial temperature of the column: 80°C, temperature rise after 8 minutes: 4.5°C/min, final temperature: 180°C. The temperature at the inlet and in the detector: 230°C.

(6) Time for analysis: 40 minutes.

(7) Quantitative method: Internal standard method using n-undecane as the internal standard substance (IS).

$C_9$ alcohol mixture (sample)/IS = 10/1 (weight ratio), amount of sample = 0.2 $\mu$l, relative mol sensitivity of alcohol = 1.0.

The $C_9$ alcohol mixture obtained as above is thus analyzed, whereupon isomers having a retention time not longer than the retention time of isobutyl n-caprylate are grouped into a first group component, isomers having a retention time longer than the retention time of isobutyl n-caprylate and not longer than the retention time of methyl n-caprate are grouped into a second group component and isomers having a retention time longer than the retention time of methyl n-caprate are grouped into a third group component.

In the present invention, the $C_9$ alcohol mixture is required to have a composition comprising from 10 to 50% by weight of the first group component, from 10 to 40% by weight of the second group component and from 30 to 70% by weight of the third group component. A more preferred composition comprises from 10 to 35% by weight of the first group component, from 10 to 40% by weight of the second group component and from 30 to 70% by weight of the third group component. A further preferred component comprises from 15 to 35% by weight of the first group component, from 10 to 40% by weight of the second group component and from 30 to 65% by weight of the third group component. A still further preferred composition comprises from 20 to 35% by weight of the first group component, from 10 to 40% by weight of the second group component and from 30 to 60% by weight of the third group component. The most preferred composition comprises from 20 to 30% by weight of the first group component, from 15 to 33% by weight of the second group component and from 35 to 60% by weight of the third group component. If the first group component is less than 8% by weight, the electric insulating properties tend to deteriorate. On the other hand, if it exceeds 50% by weight, the heat resistance and the cold resistance tend to deteriorate. Further, if the third group component is less than 20% by weight, the heat resistance and the cold resistance tend to deteriorate, and if it exceeds 80% by weight, the electrical insulating properties tend to deteriorate.

The $C_9$ alcohol mixture of the present invention thus obtained may be reacted with e.g. phthalic anhydride by a usual method for esterification and then purified by a usual method to obtain a plasticizer (such as a phthalate plasticizer).

When a vinyl chloride polymer and the above plasticizer are mixed in predetermined proportions and the properties of the plasticizer are compared with e.g. a conventional bis (2-ethylhexyl) phthalate prepared from 2-ethylhexanol, the plasticizer obtained by the present invention is excellent in both the cold resistance and the electrical insulating properties and has superior properties as a whole, although it may be inferior to some extent in the plasticizing efficiency.

Now, the present invention will be described in detail with reference to Examples. However, it should be understood that the present invention is by no means restricted to such specific Examples.

## EXAMPLE 1

### (1) Preparation of octenes

A $C_4$ fraction (composed of 6% by weight of isobutene, 43% by weight of 1-butene, 25% by weight of 2-butene, 25% by weight of butanes and 1% by weight of others) obtained by removing butadiene and isobutene from a BB fraction obtained from a cracker of naphtha, was dehydrated by molecular sieve 13X. Then, 4 kg of the dehydrated $C_4$ fraction, 5.5 g of a n-hexane solution of nickel octanoate (Ni content: 6% by weight) and 11.3 g of ethylaluminum dichloride were charged under a nitrogen atmosphere to an induction agitation-type SUS autoclave having a capacity of 10 liters, and the mixture was reacted at 40°C for 7 hours.

After the reaction, 340 g of a 5 wt% $H_2SO_4$ aqueous solution was added to the mixture to deactivate the catalyst, and then the mixture was subjected to liquid-liquid separation to obtain octenes.

The above reaction was repeated three times.

(2) Distillation to obtain an octene fraction

The octenes obtained in the above step (1) were rectified by Oldershow type distillation tower having an inner diameter of 50 mm and 20 plates under atmospheric pressure. An octene fraction having a tower top temperature of from 108 to 127°C was obtained in an amount of 5.8 kg.

(3) Preparation of C$_9$ alcohol mixture (rhodium catalyst)

Into a SUS autoclave having a capacity of 10 liters, 2.0 kg of the octene fraction obtained in the above step (2) and 1.2 g of an aqueous rhodium acetate solution (10% as rhodium metal) were charged and reacted at a reaction temeperature of 135°C while maintaining the total pressure at a level of 157 bar (160 kg/cm$^2$G) by an oxo gas having a ratio of H$_2$/CO = 1. Five hours later, the absorption of gas was no longer observed, and the reactor was cooled rapidly. The oxo gas was released, and the entire amount of the reaction solution was taken out and then subjected to distillation under reduced pressure of 10 mmHg to obtain aldehydes and alcohols. The total amount of the aldehydes and alcohols obtained was 99.2%.

Then, into a SUS autoclave having a capacity of 10 liters, the entire amount of the product obtained by the above distillation and 160 g of a nickel-supported solid catalyst were charged under a nitrogen atmosphere and reacted at a reaction temperature of 150°C while maintaining the total pressure at a level of 88 bar (90 kg/cm$^2$G) with hydrogen gas.

Five hours later, the absorption of gas completed, and the reactor was cooled rapidly. The hydrogen gas was released, and the entire amount of the reaction solution was taken out. The solid catalyst was removed by filtration, and the filtrate was rectified by Oldershow type distillation tower having an inner diameter of 35 mm and 20 plates. The reflux ratio was 10, and the pressure was 10 mmHg. By this rectification, five types of C$_9$ alcohol mixtures (R-1, R-2, R-3, R-4 and R-5) were obtained. These five types of C$_9$ alcohol mixtures were analyzed by gas chromatography under the above-mentioned analytical conditions (1) to (7). The compositions of the C$_9$ alcohol mixtures are shown in Table 1.

(4) Evaluation of the properties of the plasticizer

The five types of C$_9$ alcohol mixtures (R-1, R-2, R-3, R-4 and R-5) obtained in the above step (3) were reacted with phthalic anhydride by a usual method for esterification, and then purified by a usual method to obtain a phthalate (plasticizer).
Polyvinyl chloride (polymerization degree n = 1300): 100 PHR
Plasticizer: 50 PHR
Stabilizer (cadmium-barium stearate): 1 PHR

Then, a mixture having the above composition was subjected to rolling by a usual method and then pressed to obtain a sheet having a thickness of 1 mm. Then, test pieces were punched out from the sheet by a dumbbel die. Each test piece was examined for the plasticizer properties. The results are shown in Table 2.

EXAMPLE 2

The operation was conducted in the same manner as in EXAMPLE 1 except that the hydroformylation reaction was conducted by using a cobalt catalyst.

Namely, into a SUS autoclave having a capacity of 10 liters, 2.0 kg of the octene fraction obtained in step (2) of EXAMPLE 1 and 20 g of dicobalt octacarbonyl were charged under a nitrogen atmosphere and reacted at a temperature of from 140 to 150°C while maintaining the total pressure at a level of 157 bar (160 kg/cm$^2$G) with an oxo gas having a ratio of H$_2$/CO = 1. Two hours later, the absorption of gas ceased, and the reactor was rapidly cooled. A 3% NaOH aqueous solution was injected to deactivate the cobalt catalyst. Then, the reactor was further cooled, and the oxo gas was released. Then, the entire amount of the reaction solution was taken out and subjected to liquid-liquid separation to obtain an organic phase.

Then, the organic layer was subjected to distillation under reduced pressure of 10 mmHg to obtain aldehydes and alcohols. The total amount of the aldehydes and alcohols obtained was 99%.

Then, into a SUS autoclave having a capacity of 10 liters, the entire amount of the product obtained by the above distillation and 160 g of a nickel-supported solid catalyst were charged under a nitrogen atmosphere and reacted at a reaction temperature of 150°C while maintaining the total pressure at a level of 88 bar (90 kg/cm$^2$G) with hydrogen gas. Five hours later, the absorption of gas ceased, and the reactor was rapidly cooled. The hydrogen gas was released, and the entire amount of the reaction solution was taken out. The solid catalyst was removed by filtration, and the filtrate was rectified by Oldershow type distillation tower having an inner diameter of 35 mm and 20 plates. The reflux ratio was 10, and the pressure was 10 mmHg. By this rectification, five types of $C_9$ alcohol mixtures (C-1, C-2, C-3, C-4 and C-5) were obtained. These five types of $C_9$ alcohol mixtures were analyzed by gas chromatography under the same analytical conditions as in EXAMPLE 1. The compositions of the $C_9$ alcohol mixtures are shown in Table 1.

The $C_9$ alcohol mixtures were evaluated for the plasticizer properties in the same manner as in EXAMPLE 1(4). The results are shown in Table 2.

COMPARATIVE EXAMPLE 1

In the same manner as in EXAMPLE 1, a $C_9$ alcohol mixture was prepared, and by the rectification, four types of $C_9$ alcohol mixtures (HR-1, HR-2, HR-3 and HR-4) were obtained. Their compositions are shown in Table 1.

These $C_9$ alcohol mixtures were esterified in the same manner as in EXAMPLE 1 and then mixed with a vinyl chloride resin to examine the plasticizer properties. The results are shown in Table 2.

COMPARATIVE EXAMPLE 2

In the same manner as in EXAMPLE 2, a $C_9$ alcohol mixture was prepared, and by the rectification, four types of $C_9$ alcohol mixtures (HC-1, HC-2, HC-3 and HC-4) were obtained. Their compositions are shown in Table 1.

These $C_9$ alcohol mixtures were esterified and their plasticizer properties were examined in the same manner as in EXAMPLE 1. The results are shown in Table 2.

COMPARATIVE EXAMPLE 3

Bis(2-ethylhexyl)phthalate (DOP) was mixed with a vinyl chloride resin and its plasticizer properties were examined in the same manner as in EXAMPLE 1. The results are shown in Table 2.

## Table 1

|  | EXAMPLE 1 | | | | | EXAMPLE 2 | | | | |
|---|---|---|---|---|---|---|---|---|---|---|
|  | R-1 | R-2 | R-3 | R-4 | R-5 | C-1 | C-2 | C-3 | C-4 | C-5 |
| First group component (wt%) | 42.9 | 32.7 | 14.5 | 13.0 | 20.8 | 43.0 | 20.7 | 15.5 | 36.8 | 19.5 |
| Second group component (wt%) | 29.4 | 29.6 | 23.4 | 19.0 | 32.0 | 29.1 | 32.2 | 15.4 | 25.6 | 10.3 |
| Third group component (wt%) | 27.3 | 37.6 | 62.1 | 68.0 | 47.1 | 27.9 | 47.1 | 68.2 | 37.5 | 70.2 |

## Table 1 (continued)

|  | COMPARATIVE EXAMPLE 1 | | | | COMPARATIVE EXAMPLE 2 | | | |
|---|---|---|---|---|---|---|---|---|
|  | HR-1 | HR-2 | HR-3 | HR-4 | HC-1 | HC-2 | HC-3 | HC-4 |
| First group component (wt%) | 75.0 | 11.0 | 15.1 | 5.2 | 75.2 | 11.4 | 15.4 | 5.5 |
| Second group component (wt%) | 5.0 | 5.2 | 70.5 | 4.7 | 4.9 | 5.7 | 69.5 | 4.5 |
| Third group component (wt%) | 19.8 | 83.7 | 14.4 | 90.0 | 19.9 | 82.9 | 15.1 | 90.0 |

Table 2

|  | EXAMPLE 1 | | | | |
|---|---|---|---|---|---|
|  | R-1 | R-2 | R-3 | R-4 | R-5 |
| Tensile force $(kg/cm^2)$ | 276 | 275 | 290 | 277 | 280 |
| Elongation (%) | 361 | 358 | 330 | 328 | 340 |
| 100% modulus $(kg/cm^2)$ | 125 | 126 | 128 | 127 | 127 |
| Evaporation ($87^\circ$C for 1 day) (%) | 1.59 | 1.5 | 1.0 | 0.98 | 1.2 |
| Cold resistance (Tf) ($^\circ$C) | -23.5 | -25.1 | -28.0 | -28.8 | -27.1 |
| Insulating properties (Volume resistance) ($\times 10^{13} \Omega \cdot$ cm) | 28.0 | 22.1 | 12.5 | 10.5 | 14.0 |

Table 2 (continued)

|  | EXAMPLE 2 | | | | |
|---|---|---|---|---|---|
|  | C-1 | C-2 | C-3 | C-4 | C-5 |
| Tensile force $(kg/cm^2)$ | 282 | 270 | 277 | 276 | 275 |
| Elongation (%) | 352 | 345 | 331 | 349 | 351 |
| 100% modulus $(kg/cm^2)$ | 124 | 126 | 129 | 127 | 126 |
| Evaporation ($87^\circ$C for 1 day) (%) | 1.54 | 1.35 | 1.09 | 1.40 | 1.00 |
| Cold resistance (Tf) ($^\circ$C) | -23.6 | -27.0 | -27.7 | -24.5 | -27.2 |
| Insulating properties (Volume resistance) ($\times 10^{13} \Omega \cdot$ cm) | 29.5 | 31.0 | 10.8 | 25.5 | 14.1 |

8

### Table 2 (continued)

|  | COMPARATIVE EXAMPLE 1 | | | |
|---|---|---|---|---|
|  | HR-1 | HR-2 | HR-3 | HR-4 |
| Tensile force $(kg/cm^2)$ | 272 | 274 | 280 | 269 |
| Elongation (%) | 370 | 320 | 330 | 328 |
| 100% modulus $(kg/cm^2)$ | 123 | 128 | 129 | 130 |
| Evaporation ($87^\circ$C for 1 day) (%) | 1.80 | 0.97 | 1.20 | 0.88 |
| Cold resistance (Tf) ($^\circ$C) | -18.8 | -27.3 | -20.0 | -29.5 |
| Insulating properties (Volume resistance) $(\times 10^{13} \Omega. cm)$ | 36.8 | 8.2 | 9.8 | 4.1 |

### Table 2 (continued)

|  | COMPARATIVE EXAMPLE 2 | | | | COMPARATIVE EXAMPLE 3 |
|---|---|---|---|---|---|
|  | HC-1 | HC-2 | HC-3 | HC-4 | DOP |
| Tensile force $(kg/cm^2)$ | 269 | 280 | 275 | 277 | 269 |
| Elongation (%) | 368 | 325 | 331 | 330 | 320 |
| 100% modulus $(kg/cm^2)$ | 124 | 127 | 128 | 130 | 123 |
| Evaporation ($87^\circ$C for 1 day) (%) | 1.75 | 0.99 | 1.18 | 0.87 | 3.0 |
| Cold resistance (Tf) ($^\circ$C) | -18.0 | -27.2 | -21.5 | -29.0 | -23.0 |
| Insulating properties (Volume resistance) $(\times 10^{13} \Omega. cm)$ | 37.1 | 8.0 | 9.2 | 3.9 | 10.0 |

As is evident from the EXAMPLES, the alcohol mixtures for plasticizer of the present invention are superior to conventional alcohols for plasticizer such as 2-ethylhexanol in the cold resistance and electrical insulating properties as the alcohol material for plasticizer for e.g. a vinyl chloride polymer. The present invention provides alcohol mixtures for plasticizer which are very useful from the industrial point of view.

EP 0 278 407 B1

**Claims**

1. A $C_9$ alcohol mixture for plasticizer obtained by the hydroformylation and hydrogenation of a $C_8$ olefin mixture as the starting material obtained by the dimerization of a butene fraction, said alcohol mixture comprising from 10 to 50% by weight of a first group component having a retention time not longer than the retention time of isobutyl n-caprylate, from 10 to 40% by weight of a second group component having a retention time longer than that of isobutyl n-caprylate and not longer than that of methyl n-caprate and from 30 to 70% by weight of a third group component having a retention time longer than that of methyl n-caprate, as divided into such three components by gas chromatography using a capillary column packed with a polyethylene glycol having a number average molecular weight of 15,000 to 20,000 as an isomer separating agent.

2. The alcohol mixture according to Claim 1, which comprises from 10 to 35% by weight of the first group component, from 10 to 40% by weight of the second group component and from 30 to 70% by weight of the third group component.

3. The alcohol mixture according to Claim 1, which comprises from 15 to 35% by weight of the first group component, from 10 to 40% by weight of the second group component and from 30 to 65% by weight of the third group component.

4. The alcohol mixture according to Claim 1, which comprises from 20 to 35% by weight of the first group component, from 10 to 40% by weight of the second group component and from 30 to 60% by weight of the third group component.

5. The alcohol mixture according to Claim 1, which comprises from 20 to 30% by weight of the first group component, from 15 to 33% by weight of the second group component and from 35 to 60% by weight of the third group component.

6. The alcohol mixture according to Claim 1, wherein the hydroformylation is conducted by means of a rhodium catalyst.

7. The alcohol mixture according to Claim 6, wherein the hydroformylation is conducted at a rhodium concentration of from 0.1 to 1,000 ppm as rhodium atom at a temperature of from 80 to 200°C under a pressure of from atmospheric pressure to 490 bar (500 kg/cm$^2$G) and a $H_2$/CO volume ratio of from 0.5 to 4.

**Patentansprüche**

1. $C_9$-Alkoholgemisch für Weichmacher, erhalten durch die Hydroformylierung und Hydrierung eines $C_8$-Olefingemisches als dem Ausgangsmaterial, das erhalten ist durch die Dimerisierung einer Butenfraktion, wobei das Alkoholgemisch von 10 bis 50 Gew.-% einer ersten Gruppenkomponente mit einer Retentionszeit, die nicht länger als die Retentionszeit von Isobutyl-n-caprylat ist, von 10 bis 40 Gew.-% einer zweiten Gruppenkomponente mit einer Retentionszeit, die länger als die von Isobutyl-n-caprylat und nicht länger als die von Methyl-n-caprinat ist und von 30 bis 70 Gew.-% einer dritten Gruppenkomponente mit einer Retentionszeit, die länger als die von Methyl-n-caprinat ist, umfaßt, wie es durch Gaschromatographie unter Verwendung einer Kapillarsäule, gepackt mit einem Polyethylenglykol mit einem Zahlenmittel des Molekulargewichtes von 15.000 bis 20.000 als einem Mittel zum Trennen der Isomeren in solche drei Komponenten aufgeteilt ist.

2. Alkoholgemisch nach Anspruch 1, das von 10 bis 35 Gew.-% der ersten Gruppenkomponente, von 10 bis 40 Gew.-% der zweiten Gruppenkomponente und von 30 bis 70 Gew.-% der dritten Gruppenkomponente umfaßt.

3. Alkoholgemisch nach Anspruch 1, das von 15 bis 35 Gew.-% der ersten Gruppenkomponente, von 10 bis 40 Gew.-% der zweiten Gruppenkomponente und von 30 bis 65 Gew.-% der dritten Gruppenkomponente umfaßt.

10

**4.** Alkoholgemisch nach Anspruch 1, das von 20 bis 35 Gew.-% der ersten Gruppenkomponente, von 10 bis 40 Gew.-% der zweiten Gruppenkomponente und von 30 bis 60 Gew.-% der dritten Gruppenkomponente umfaßt.

**5.** Alkoholgemisch nach Anspruch 1, d von 20 bis 30 Gew.-% der ersten Gruppenkomponente, von 15 bis 33 Gew.-% der zweiten Gruppenkomponente und von 35 bis 60 Gew.-% der dritten Gruppenkomponente umfaßt.

**6.** Alkoholgemisch nach Anspruch 1, worin die Hydroformylierung mittels eines Rhodium-Katalysators ausgeführt ist.

**7.** Alkoholgemisch nach Anspruch 6, worin die Hydroformulierung bei einer Rhodium-Konzentration von 0,1 bis 1.000 ppm als Rhodiumatome, bei einer Temperatur von 80 bis 200 °C unter einem Druck vom atmosphärischen Druck bis 490 bar (500 kg/cm$^2$G) und einem Volumenverhältnis von H$_2$/CO von 0,5 bis 4 ausgeführt ist.

**Revendications**

**1.** Mélange d'alcools en C$_9$ pour plastifiant, obtenu par l'hydroformylation et l'hydrogénation d'un mélange d'oléfines en C$_8$ en tant que matière de départ, obtenu par la dimérisation d'une fraction butène, ledit mélange d'alcools comprenant de 10 à 50% en poids d'un composant de premier groupe ayant un temps de rétention non supérieur au temps de rétention du n-caprylate d'isobutyle, de 10 à 40% en poids d'un composant de second groupe ayant un temps de rétention supérieur à celui du n-caprylate d'isobutyle et non supérieur à celui du n-caprate de méthyle, et de 30 à 70% en poids d'un composant de troisième groupe ayant un temps de rétention supérieur à celui du n-caprate de méthyle, tel que séparé en ces trois composants par chromatographie en phase gazeuse à l'aide d'une colonne capillaire garnie d'un polyéthylène glycol ayant une masse moléculaire moyenne en nombre de 15 000 à 20 000 en tant qu'agent de séparation d'isomères.

**2.** Mélange d'alcools selon la revendication 1, qui comprend de 10 à 35% en poids du composant de premier groupe, de 10 à 40% en poids du composant de second groupe, et de 30 à 70% en poids du composant de troisième groupe.

**3.** Mélange d'alcools selon la revendication 1, qui comprend de 15 à 35% en poids du composant de premier groupe, de 10 à 40% en poids du composant de second groupe, et de 30 à 65% en poids du composant de troisième groupe.

**4.** Mélange d'alcools selon la revendication 1, qui comprend de 20 à 35% en poids du composant de premier groupe, de 10 à 40% en poids du composant de second groupe, et de 30 à 60% en poids du composant de troisième groupe.

**5.** Mélange d'alcools selon la revendication 1, qui comprend de 20 à 30% en poids du composant de premier groupe, de 15 à 33% en poids du composant de second groupe, et de 35 à 60% en poids du composant de troisième groupe.

**6.** Mélange d'alcools selon la revendication 1, dans lequel l'hydroformylation est conduite à l'aide d'un catalyseur au rhodium.

**7.** Mélange d'alcools selon la revendication 6, dans lequel l'hydroformylation est conduite à une concentration en rhodium de 0,1 à 1000 ppm en tant que rhodium atomique, à une température de 80 à 200 °C, sous une pression allant de la pression atmosphérique à 490 bars (500 kg/cm$^2$ au manomètre), et avec un rapport en volume H$_2$/CO de 0,5 à 4.